**Europäisches Patentamt**

(19) **European Patent Office**

**Office européen des brevets**

(11) Publication number : **0 394 026 B1**

# (12) EUROPEAN PATENT SPECIFICATION

(45) Date of publication of patent specification :
**29.09.93 Bulletin 93/39**

(51) Int. Cl.⁵ : **A61K 31/47, A61K 9/08**

(21) Application number : **90304182.0**

(22) Date of filing : **19.04.90**

(54) Formulation containing an imidazo[4,5-c]quinolin derivative.

The file contains technical information submitted after the application was filed and not included in this specification

(30) Priority : **20.04.89 US 341492**

(43) Date of publication of application :
**24.10.90 Bulletin 90/43**

(45) Publication of the grant of the patent :
**29.09.93 Bulletin 93/39**

(84) Designated Contracting States :
**BE CH DE DK ES FR GB IT LI NL SE**

(56) References cited :
**EP-A- 376 534**
**EP-A- 0 219 784**
**US-A- 4 640 930**
**US-A- 4 689 338**

(73) Proprietor : **RIKER LABORATORIES, INC.**
**P.O. Box 33427**
**Saint Paul Minnesota 55133-3427 (US)**

(72) Inventor : **Schultz, Helen Jensen, c/o Riker Laboratories Inc.**
**2501 Hudson Road, P.O. Box 33427**
**St. Paul, MN 55133-3427 (US)**

(74) Representative : **Baillie, Iain Cameron et al**
**c/o Ladas & Parry, Altheimer Eck 2**
**D-80331 München (DE)**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 394 026 B1

## Description

This invention pertains to pharmaceutical formulations suitable for the parenteral administration of drugs. More particularly, it pertains to solutions containing 1-isobutyl-IH-imidazo[4,5-c]quinolin-4-amine or 1-(2-hydroxy-2-methylpropyl)-1-H-imidazo[4,5-c]quinolin-4-amine which are suitable for parenteral administration.

The compound 1-isobutyl-IH-imidazo[4,5-c]quinolin-4-amine and pharmaceutically acceptable acid addition salts such as the hydrochloride salt are disclosed in U.S. Patent No. 4,689,338 and described therein as an antiviral agent and as an interferon inducer. A variety of formulations for topical administration are described but a formulation suitable for parenteral administration of this compound is not disclosed. U.S Patent No. 4,689,338 also similarly discloses the compound 1-(2-hydroxy-2-methylpropyl)-IH-imidazo[4,5-c]quinolin-4-amine.

U.S. Patent Nos. 4,640,930, 4,652,561 and 4,808,580 describe parenteral solutions containing (+/-)-cis-3-(acetyloxy)-2,3-dihydro-5-[2-(dimethylamino)ethyl]-2-(4-methoxyphenyl)naphtho[1,8-bc]-1,5-thiazocin-4(5H)-one hydrochloride, (+/-)-cis-3-(acetyloxy)-2,3-dihydro-2-(4-methoxyphenyl)-5-[2-(dimethylamino)ethyl]naptho[1,2-b]-1-4-thiazepin-4(5H)-one hydrochloride, and cis-rac-2,3-dihydro-3-hydroxy-5-[2-(dimethylamino)ethyl]2-(4-methoxyphenyl)-naphtho[2,1-2b][1,4-thiazepin-4(5H)-one hydrochloride, respectively in benzyl alcohol, sorbitol, hydrochloric acid, sodium hydroxide and water.

European Patent Application No 376 534 which is available pursuant to Article 54(3) EPC for the purpose of absolute verbal anticipation only discloses pharmaceutical formulations and adhesive coated sheet materials for the topical and/or transdermal delivery of 1-isobutyl-1H-imidazo [4,5-c]quinolin-4-amine. However the disclosed pharmaceutical compositions differ in both chemical composition and relevant physical properties so as to be unsuitable for administration by injection.

The present invention provides an aqueous solution suitable for the parenteral administration comprising 1-isobutyl-1H-imidazo[4,5-c]quinolin-4-amine or 1-(2-hydroxy-2-methylpropyl)-IH-imidazo[4,5-c]quinolin-4-amine dissolved in an aqueous acid, the acid being selected from hydrochloric acid, lactic acid, acetic acid, aspartic acid and a mixture of two or more of the foregoing, and further comprising a tonicity adjuster selected from glycerin, sorbitol and dextrose in an amount such that the osmolality of said solution is between 235 and 335 mOsm/kg, the solution being further characterized in that it has a pH of between 2 and 6.

A solution of the invention can be used for the parenteral administration of 1-isobutyl-1H-imidazo[4,5-c]-quinolin-4-amine or 1-(2-hydroxy-2-methylpropyl)-1H-imidazo [4,5-c]quinolin-4-amine. The solution may be administered by intramuscular injection, intradermal injection, subcutaneous injection or intravenous injection.

The present invention provides aqueous solutions suitable for parenteral administration that contain 1-isobutyl-IH-imidazo[4,5-c]quinolin-4-amine or 1-(2-hydroxy-2-methylpropyl)-1-H-imidazo[4,5-c]quinolin-4- amine dissolved in an aqueous acid such as hydrochloric acid, lactic acid, acetic acid, aspartic acid or mixtures thereof.

The compounds I-isobutyl-IH-imidazo[4,5-c]quinolin-4-amine and 1-(2-hydroxy-2-methylpropyl)-IH-imidazo[4,5-c]-quinolin-4-amine are known antiviral agents that are also known to induce interferon biosynthesis. They can be prepared using the method disclosed in U.S. Patent No. 4,689,338, The compounds can be used to treat viral infections such as Type I or Type II Herpes simplex infections and genital warts. Furthermore, the fact that the compounds are interferon inducers suggests that they, and therefore solutions containing them, might be useful in the treatment of numerous other diseases, such as rheumatoid arthritis, warts, eczema, hepatitis B, psoriasis, multiple sclerosis, essential thrombocythaemia, and cancer, such as basal cell carcinoma and other neoplastic diseases. The amount of 1-isobutyl-1H-imidazo[4,5-c]quinolin-4-amine or 1-(2-hydroxy-2-methylpropyl)-1H-imidazo[4,5-c]quinolin-4-amine present in a solution of the invention will be an amount effective to treat the targeted disease state, to prevent recurrence of such a disease or to promote immunity against such a disease. The amount is preferably from 0.01 milligrams to 15 milligrams, more preferably from 4 milligrams to 12 milligrams, per milliliter of the solution.

An acid selected from hydrochloric acid, lactic acid, acetic acid, aspartic acid or mixtures thereof is incorporated into a solution of the invention. The currently preferred acid is hydrochloric acid. The acid will be present in amount sufficient to keep the 1-isobutyl-IH-imidazo[4,5-c]quinolin-4-amine or 1-(2-hydroxy-2-methylpropyl)-1H-imidazo[4,5-c]quinolin-4-amine in solution. The acid will generally be present in an amount of between 0.5 moles and 20 moles of acid per mole of 1-isobutyl-1H-imidazo[4,5-c]quinolin-4-amine or 1-(2-hydroxy-2-methylpropyl)-1-H-imidazo[4,5-c]quinolin-4-amine. Preferably the molar ratio of acid to 1-isobutyl-IH-imidazo[4,5-c]quinolin-4-amine or 1-(2-hydroxy-2-methylpropyl)-1H-imidazo[4,5-c]quinolin-4-amine will be between 1/1 and 4/1.

The acid may be provided through the presence of the respective acid-addition salt of 1-isobutyl-1H-imidazo[4,5-c]-quinolin-4-amine or 1-(2-hydroxy-2-methylpropyl)-1H-imidazo [4,5-c]quinolin-4-amine. For example, an aqueous hydrochloric acid solution may be obtained by using 1-isobutyl-1H-imidazo[4,5-c]quinolin-4- amine hydrochloride in place of the free base. Additional acid such as hydrochloric acid may also be added even when an acid-addition salt of 1-isobutyl-1H-imidazo[4,5-c]quinolin-4-amine or 1-(2-hydroxy-2-methylpropyl)-1H-imidazo[4,5-c]-quinolin-4-amine is employed.

In order to minimize pain on injection and tissue irritation it is desirable that the pH of the solutions of the invention not be below 2. The pH of the solution will generally be between 2 and 6. Optionally, a solution of the invention may further comprise a base to provide the desired pH. It is preferred that the pH be between about 2.5 and 4.5. The currently preferred base is sodium hydroxide. Care must be taken in adjusting the pH of the solutions so that the 1-isobutyl-lH-imidazo[4,5-c]quinolin-4-amine or 1-(2-hydroxy-2-methylpropyl)-lH-imidazo[4,5-c]quinolin-4-amine does not precipitate.

An additional factor affecting pain on injection and tissue irritation at the injection site is the osmoticity of the solution. It is preferred that the solution be isotonic with serum or isoosmotic with 0.9% sodium chloride. A quantitative term used to state osmotic properties is the osmol. An osmol is defined as the weight in grams of a solute, existing in a solution as molecules (and/or ions, macromolecules, aggregates, etc), that is osmotically equivalent to the gram-molecular-weight of an ideally behaving nonelectrolyte. A milliosmol, abbreviated mOsm, is the weight stated in milligrams. A solution has an osmolal concentration of one when it contains one osmol of solute per kilogram of water. A solution has an osmolality of n when it contains n osmols per kilogram of water. Further discussion of tonicity, osmoticity and isomolality may be found in Chapter 80 in the Seventeenth Edition of Remington's Pharmaceutical Sciences,. A range of values from 275 mOsm/kg to 305 mOosm/kg has been reported for serum osmolality. Accordingly, preferred solutions of the invention may further comprise a tonicity adjuster present in an amount such that the osmolality of the solution is between 235 mOsm/kg to 335 mOsm/kg and most preferably between 270 mOsm/kg and 310 mOsm/kg. The osmolality of a solution is determined using the test method described below. Care must be taken in the choice of tonicity adjusters so that the 1-isobutyl-lH-imidazo[4,5-c]quinolin-4-amine or 1-(2-hydroxy-2-methylpropyl)-1H-imidazo[4,5-c]quinolin-4-amine stays in solution. The currently preferred tonicity adjusters are sorbitol and glycerin. When sorbitol is used it will generally be present in an amount of about 40 milligrams per milliliter of solution.

A preferred solution in accordance with the invention can be prepared by combining the acid with a major portion of the water to be used in the formulation, adding the 1-isobutyl-lH-imidazo[4,5-c]quinolin-4-amine or 1-(2-hydroxy-2-methylpropyl)-1H-imidazo[4,5-c]quinolin-4-amine to this solution and stirring until all material is in solution. The sorbitol may then be added and stirring continued until dissolution is complete. The pH of the solution is then measured and a base may be added to adjust the pH if desired. A sufficient quantity (qs) of water is then added to bring the solution to the desired total volume. The bulk solution may then be filtered through a 0.2 micrometers filter, heat sealed into USP Type I glass ampules and autoclaved.

Preferred solutions of the invention may be stored at room temperature for a period of at least 30 days without precipitation or degradation of the active ingredient. Most preferred solutions exhibit such stability for a period of at least one year.

The following test methods have been employed in the examples which thereafter follow.

Osmolality Determination

Osmolality was determined using a Molecular Weight Apparatus, Model 233 available from Wescan Instruments. A series of 5 standard solutions of sodium chloride in water having known osmolality values were prepared. Microvolt readings for each standard were taken on the Molecular Weight Apparatus. Using linear regression analysis, the readings together with the known osmolality values were used to determine the values of m and b in the equation below.

$$\text{instrument reading} = m(\text{osmolality in mOsm/kg}) + b$$

The values of m and b in conjunction with the instrument readings for the solutions of the invention allow the osmolality values of the solutions to be calculated. The readings for the standards and the solutions of the invention were preferably taken on the same day to insure accuracy. At least three readings were taken for each standard and solution and the average was used in the above calculations.

The above method is to be used for purposes of construing the claims, with the number of readings being taken being five.

Determination of 1-Isobutyl-lH-imidazo[4,5-c]quinolin-4-amine Content

The solutions used in the stability studies were analyzed for 1-isobutyl-lH-imidazo[4,5-c]quinolin-4-amine content by conventional high pressure liquid chromatography as follows:

A 15 centimeter by 4 millimeter column containing Zorbax® $C_8$ (an octylsilane available from E. I. DuPont de Nemours & Company), 5 micrometers particle size, was used. The mobile phase was 35 percent acetonitrile/65 percent water (volume/volume) containing 0.2 percent tetramethylammonium hydroxide and 0.2 percent 1-dodecanesulfonate sodium, with the pH of the mobile phase adjusted to 2.0 with phosphoric acid. The flow rate was 2.5 ml per minute. Ultraviolet detection at 254 nanometers was used.

The following examples are provided to illustrate the invention, but are not intended to be limiting thereof.

Example 1

A mixture of 0.20 g 1-isobutyl-lH-imidazo[4,5-c]quinolin-4-amine, 0.20 g of 85% lactic acid, 0.344 g of glycerin and 19.256 g of water was placed in a glass vial and shaken until all material was dissolved. The resulting solution of the invention had a pH of 3.59 and the osmolality was 279 mOsm/Kg. No drug precipitated on storage at room temperature for 30 days.

Example 2

A mixture of 0.20 g of 1-isobutyl-lH-imidazo[4,5-c]quinolin-4-amine, 0.20 g of 85% lactic acid, 1.10 g of sorbitol 50% (prepared by combining 29 ml of water and 71 ml of sorbitol 70%) and 18.50 g of water was placed in a glass vial and shaken until all material was dissolved. The resulting solution of the invention had a pH of 3.58 and the osmolality was 271 mOsm/Kg. No drug precipitated on storage at room temperature for 30 days.

Example 3

A mixture of 0.050 g of 1-isobutyl-lH-imidazo[4,5-c]quinolin-4-amine, 22 ml of 0.lN HCl and 15 ml of water was placed in a beaker and stirred until all material was in solution. To this solution was added 2.0 g of sorbitol and stirring was continued until the sorbitol was dissolved. The solution was transferred to a 50 ml volumetric flask. The pH of the solution was adjusted with 2N sodium hydroxide then the flask was filled to the mark with water. The compositon of the final formulation of the invention is shown in Table 1.

Examples 4-6

Using the general method of Example 3, the formulations of the invention shown in Table 1 were prepared.

## Table 1

|  | Ex. 3 | Ex. 4 | Ex. 5 | Ex. 6 |
|---|---|---|---|---|
| 1-Isobutyl-1H-imidazo-[4,5-c]quinolin-4-amine (mg/ml) | 1.0 | 2.5 | 5.0 | 10.0 |
| Sorbitol (mg/ml) | 40 | 40 | 40 | 40 |
| 0.1N HCl (ml/ml) | 0.44 | 0.44 | 0.44 | 0.44 |
| 2N NaOH qs to pH | 3.5 | 3.2 | 3.2 | 3.2 |
| Water qs to 1 ml Osmolality (mOsm/Kg) | 303 | 307 | 294 | 284 |

Example 7

To a 500 ml volumetric flask was added 5.00 g of 1-isobutyl-IH-imidazo[4,5-c]quinolin-4-amine, 200 ml of 0.IN hydrochloric acid and 230 ml of sterile water. The flask was sonicated for 50 minutes then an additional 20 ml of 0.IN hydrochloric acid was added and sonicating was continued for 20 minutes. The flask was heated on a steam bath to 62°C then sonicated for 30 minutes. A small amount of insoluble material remained which was removed during the filtration step below. The flask was allowed to cool to 25°C before 20.0 g of sorbitol was added. The sorbitol was mixed by inverting the flask by hand until the sorbitol had dissolved. The pH was adjusted by adding 1.35 ml of 2N sodium hydroxide. A quantity of sterile water sufficient to bring the final volume to 500 ml was added. The formulation was filtered through a 0.2 micrometers filter then heat sealed in 2 ml Type I clear glass ampules. The ampules were autoclaved at 121°C for 30 minutes. The ampules were divided into groups and stored under a variety of conditions. At selected time points ampules were evaluated for color and clarity of solution, pH and 1-isobutyl-IH-imidazo[4,5-c]quinolin-4-amine content. The results relating to this formulation of this invention are summarized in Table 2.

## Table 2

| Storage Condition | Time | pH | 1-Isobutyl-1H-imidazo[4,5-c]-quinolin-4-amine content (mg/ml) |
|---|---|---|---|
| initial | | 3.1 | 9.94 |
| 50°C | 4 weeks | 3.6 | 9.93 |
| 50°C | 8 weeks | 3.4 | 10.5 |
| 50° | 12 weeks | 3.5 | 9.94 |
| 40°C | 4 weeks | 3.6 | 9.88 |
| 40°C | 8 weeks | 3.5 | 10.3 |
| 40°C | 12 weeks | 3.4 | 9.92 |
| 40°C | 6 months | 3.2 | 10.8 |
| 30°C | 4 weeks | 3.5 | 9.92 |
| 30°C | 12 weeks | 3.4 | 9.95 |
| 30°C | 6 months | 3.3 | 10.6 |
| 30°C | 9 months | 3.2 | 10.5 |
| 30°C | 12 months | 3.4 | 10.1 |
| 20°C* | 2 weeks | 3.3 | 9.81 |
| 20°C* | 4 weeks | 3.5 | 9.86 |
| 20°C* | 8 weeks | 3.4 | 10.2 |
| 20°C* | 12 weeks | 3.4 | 9.95 |
| Freeze-thaw 3 cycles | | 3.4 | 9.96 |

* with 30,5 m (100 foot) candle light

Throughout the study, all ampules contained clear, colorless solutions.

EP 0 394 026 B1

### Example 8

To a glass carboy was added 0.15 g of 1-isobutyl-lH-imidazo[4,5-c]quinolin-4-amine, 220 ml of lN hydrochloric acid and 4200 ml of sterile water. The carboy was mixed until all material was dissolved, then 200 g of sorbitol was added and swirled until dissolved. The pH was adjusted by adding 112.1 ml of 2N sodium hydroxide. A sufficient quantity of sterile water was added to bring the total volume of formulation to 5000 ml. The formulation was mixed with a magnetic stir bar then filtered through two 0.22 micrometers filters. The filtrate was heat sealed into Type I 10 ml clear glass ampules. The ampules were autoclaved at 121°C for 30 minutes. The composition of the final formulation of the invention is shown in Table 3. Stability data is shown in Table 4.

### Examples 9-11

Using the general method of Example 8, formulations having the compositions shown in Table 3 were prepared. The stability data is shown in Table 4.

### Table 3

|  | Ex. 8 | Ex. 9 | Ex. 10 | Ex. 11 |
|---|---|---|---|---|
| 1-Isobutyl-lH-imidazo-[4,5-c]quinolin-4-amine (mg/ml) | 0.03 | 0.15 | 0.9 | 3.0 |
| Sorbitol NF (mg/ml) | 40 | 40 | 40 | 40 |
| lN HCl (ml/ml) | 0.044 | 0.044 | 0.044 | 0.044 |
| 2N NaOH qs to pH | 3.1 | 3.4 | 3.3 | 3.5 |
| Sterile water qs to 1 ml | | | | |

6

TABLE 4

| Storage Condition | Time | pH | Ex. 8 Content | pH | Ex. 9 Content | pH | Ex. 10 Content | pH | Ex. 11 Content |
|---|---|---|---|---|---|---|---|---|---|
| Initial | | 3.25 | 0.0296 | 3.48 | 0.150 | 3.39 | 0.945 | 3.60 | 3.06 |
| 50°C | 4 wk | 3.28 | 0.0302 | *** | | *** | | 3.64 | 3.05 |
| 40°C | 12 wk | 3.33 | 0.0299 | *** | | *** | | 3.85 | 3.09 |
| 30°C | 4 wk | 3.27 | 0.0297 | 3.56 | 0.151 | 3.34 | 0.934 | 3.7 | 3.04 |
| 30°C | 12 wk | 3.33 | 0.0299 | 3.56 | 0.151 | 3.46 | 0.931 | 3.63 | 3.04 |
| 30°C | 6 mon | 3.28 | 0.0299 | 3.57 | 0.148 | 3.48 | 0.919 | 3.83 | 2.98 |
| 30°C | 9 mon | 3.36 | 0.0289 | 3.62 | 0.146 | 3.52 | 0.904 | 3.82 | 2.86 |
| 20°C* | 4 wk | 3.27 | 0.0293 | 3.56 | 0.150 | 3.31 | 0.933 | 3.7 | 3.12 |
| 20°C* | 12 wk | 3.30 | 0.0282 | 3.66 | 0.148 | 3.44 | 0.935 | 3.61 | 3.06 |
| 20°C** | 4 wk | 3.26 | 0.0248 | 3.51 | 0.144 | 3.25 | 0.935 | 3.8 | 3.07 |

Content - amount of 1-isobutyl-1H-imidazo[4,5-c]quinolin-4-amine (mg/ml)
* with 30,5 m (100 foot) candle light
** with 30,5 m (1000 foot) candle light
*** not available

EP 0 394 026 B1

### Example 12

A 6 liter flask was charged with 220.0 ml of 1.0N hydrochloric acid and approximately 4200 ml of sterile water; then 25.0 gram of 1-isobutyl-lH-imidazo[4,5-c]quinolin-4-amine was added and the resulting mixture stirred with a magnetic stir bar until all material was dissolved. To this solution was added 200.0 gram of sorbitol and stirring was continued until the sorbitol was dissolved. The pH of the solution was adjusted to pH 3.32 by adding 62.3 ml of 2N sodium hydroxide. Sterile water was added in a quantity sufficient to bring the total volume to 5.0 liters. The solution was filtered through a 0.22 micrometers filter then sealed in 2 ml Type I glass ampules. The ampules were autoclaved at 121°C for 30 minutes. The composition of the final formulation of the invention is shown in Table 5. Stability data is shown in Table 6.

### Example 13

Using the general method of Example 12, the formulation of the invention shown in Table 5 was prepared. The stability data is shown in Table 6.

## Table 5

|  | Example 12 | Example 13 |
|---|---|---|
| 1-Isobutyl-lH-imidazo-[4,5-c]quinolin-4-amine (mg/ml) | 5.00 | 10.00 |
| Sorbitol NF (mg/ml) | 40 | 40 |
| 1.0N HCl (ml/ml) | 0.044 | 0.044 |
| 2N NaOH qs to pH | 3.32 | 3.15 |
| Sterile water qs to 1 ml |  |  |

## Table 6

| Storage | | Example 12 | | Example 13 | |
| Condition | Time | pH | Content | pH | Content |
|---|---|---|---|---|---|
| Initial | | 3.4 | 4.98 | 3.2 | 9.85 |
| 50°C | 4 weeks | 3.1 | 4.98 | 3.1 | 9.77 |
| 50°C | 12 weeks | 3.3 | 4.99 | 3.2 | 9.91 |
| 30°C | 12 weeks | 3.2 | 5.02 | 3.1 | 9.85 |
| 20°C* | 4 weeks | 3.2 | 4.96 | 3.1 | 9.79 |
| 20°C* | 12 weeks | 3.2 | 4.99 | 3.1 | 9.94 |
| 20°C** | 4 weeks | 3.2 | 4.96 | 3.1 | 9.83 |

Content = amount of 1-isobutyl-1H-imidazo[4,5-c]quinolin-4-amine (mg/ml)

\* with 30,5 m (100 foot) candle light

\*\* with 305 m (1000 foot) candle light

Example 14

A mixture of 0.23 g of 1-isobutyl-1H-imidazo [4,5-c]quinolin-4-amine hydrochloride, 0.40 g of glycerin and 19.34 g of sterile water was placed in a glass vial and shaken until all material was dissolved. The resulting solution of the invention had a pH of 4.11 and the osmolality was 280 mOsm/kg.

Example 15

A mixture of 0.23 g of 1-isobutyl-1H-imidazo [4,5-c]quinolin-4-amine hydrochloride, 1.50 g of sorbitol 50% (prepared by combining 29 ml of water and 71 ml of sorbitol 70%) and 18.27 g of sterile water was placed in a glass vial and shaken until all material was dissolved. The resulting solution of the invention had a pH of 4.08 and the osmolality was 298 mOsm/kg.

Example 16

An aqueous solution was prepared from the following ingredients:

1-(2-hydroxy-2-methylpropyl)-1H-imidazo[4,5-c]quinolin-4-amine     0.2g

Lactic acid (85% in water)     0.19g

Sterile water qs to 25 ml

The pH of the above solution was 3.3.

It is believed that the tonicity of the solution could be modified through inclusion of, for example, sorbitol in an appropriate amount to provide a solution of the invention.

## Claims

### Claims for the following Contracting States : BE, CH, DE, FR, GB, IT, LI, NL SE

1. An aqueous solution of 1-isobutyl-IH-imidazo[4,5-c]quinolin-4-amine or 1-(2-hydroxy-2-methylpropyl)-1-H-imidazo[4,5-c]quinolin-4-amine suitable for parenteral administration comprising 1-isobutyl-IH-imidazo[4,5-c]-quinolin-4-amine or 1-(2-hydroxy-2-methylpropyl)-1H-imidazo[4,5-c]quinolin-4-amine dissolved in an aqueous acid, said acid selected from hydrochloric acid, lactic acid, acetic acid, aspartic acid and a mixture of two or more of the foregoing, and further comprising a tonicity adjuster selected from glycerin, sorbitol and dextrose in an amount such that the osmolality of said solution is between 235 and 335 mOsm/kg, said solution being further characterized in that it has a pH of between 2 and 6.

2. A solution according to claim 1, wherein 1-isobutyl-1H-imidazo[4,5-c]quinolin-4-amine or 1-(2-hydroxy-2-methylpropyl)-1H-imidazo[4,5-c]quinolin-4-amine is present in an amount effective for the intended therapeutic purpose.

3. A solution according to Claim 1, wherein said wherein said acid is hydrochloric acid.

4. A solution according to Claim 3, comprising 1-isobutyl-1H-imidazo[4,5-c]quinolin-4-amine and wherein said hydrochloric acid is provided at least in part by hydrochloric acid present in 1-isobutyl-1H-imidazo[4,5-c]quinolin-4-amine hydrochloride.

5. A solution according to Claim 1, wherein said wherein said acid is lactic acid.

6. A solution according to any preceding claim, wherein the pH is between 2.5 and 4.5.

7. A solution according to any preceding claim, comprising said 1-isobutyl-1-H-imidazo[4,5-c]quinolin-4-amine or 1-(2-hydroxy-2-methylpropyl)-H-imidazo[4, 5-c]quinolin-4-amine in an amount of 0.01 to 15 milligrams per milliliter of said solution.

8. A solution according to Claim 7, wherein said 1-isobutyl-1-H-imidazo[4,5-c]quinolin-4-amine or 1-(2-hydroxy-2-methylpropyl)-1-H-imidazo[4,5-c]quinolin-4-amine is present in an amount of 4 to 12 milligrams per milliliter of said solution.

9. A solution according to any preceding claim, further comprising a base.

10. A solution according to Claim 9, wherein said base is sodium hydroxide.

11. A solution according to any preceding claim, wherein said tonicity adjuster is sorbitol.

12. A solution according to any preceding claim, wherein the molar ratio of said acid to said 1-isobutyl-1H-imidazo-[4,5-c]quinolin-4-amine or 1-(2-hydroxy-2-methylpropyl)-1H-imidazo[4, 5-c]quinolin-4-amine is between 0.5/1 and 20/1.

13. A solution according to Claim 12, wherein the molar ratio of said acid to said 1-isobutyl-1H-imidazo-[4,5-c]quinolin-4-amine or 1-(2-hydroxy-2-methylpropyl)-1H-imidazo[4,5-c]quinolin-4-amine is between 1/1 and 4/1.

14. A solution according to any preceding claim, wherein the osmolality of said solution is between 270 and 310 mOsm/kg.

### Claims for the following Contracting State : ES

1. A method of preparing an aqueous solution of 1-isobutyl-1H-imidazo[4,5-c]quinolin-4-amine or 1-(2-hydroxy-2-methyl-propyl)-1H-imidazo[4,5-c]quinolin-4-amine suitable for parenteral administration comprising dissolving 1-isobutyl-1H-imidazo[4,5-c]-quinolin-4-amine or 1-(2-hydroxy-2-methyl-propyl)-1H-imidazo[4,5-c]quinolin-4-amine in an aqueous acid, said acid selected from hydrochloric acid, lactic acid, acetic acid, aspartic acid and a mixture of two or more of the foregoing, and further adding a tonicity adjuster selected from glycerin, sorbitol and dextrose in an amount such that the osmolality of said solution is between 235 and 335 mOsm/kg, said solution being adjusted to have a pH of between 2 and 6.

2. A method according to claim 1, wherein 1-isobutyl-1H-imidazo[4,5-c]quinolin-4-amine or 1-(2-hydroxy-2-methylpropyl)-1H-imidazo[4,5-c]quinolin-4-amine is added in an amount effective for the intended therapeutic purpose.

3. A method according to Claim 1 or Claim 2, wherein said acid is hydrochloric acid.

4. A method according to Claim 3, comprising 1-isobutyl-1H-imidazo[4,5-c]quinolin-4-amine and wherein said hydrochloric acid is provided at least in part by hydrochloric acid present in 1-isobutyl-1H-imidazo-[4,5-c]quinolin-4-amine hydrochloride.

5. A method according to Claim 1 or Claim 2, wherein said acid is lactic acid.

6. A method according to any preceding claim wherein the pH is between 2.5 and 4.5.

7. A method according to any preceding claim comprising said 1-isobutyl-1H-imidazo[4,5-c]quinolin-4-amine or 1-(2-hydroxy-2-methylpropyl)-1H-imidazo[4,5-c]quinolin-4-amine added in an amount of 0.01 to 15 milligrams per milliliter of said solution.

8. A method according to Claim 7, wherein said 1-isobutyl-lH-imidazo[4,5-c]quinolin-4-amine or 1-(2-hydroxy-2-methylpropyl)-1-H-imidazo[4,5-c]quinolin-4-amine is present in an amount of 4 to 12 milligrams per milliliter of said solution.

9. A method according to Claim 1, further comprising adding a base.

10. A method according to Claim 9, wherein said base is sodium hydroxide.

11. A method according to any preceding claim, wherein said tonicity adjuster is sorbitol.

12. A method according to any preceding claim wherein the molar ratio of said acid to said 1-isobutyl-1H-imidazo-[4,5-c]quinolin-4-amine or 1-(2-hydroxy-2-methylpropyl)-1H-imidazo[4,5-c]quinolin-4-amine is between 0.5/1 and 20/1.

13. A method according to Claim 12 wherein the molar ratio of said acid to said 1-isobutyl-lH-imidazo-[4,5-c]quinolin-4-amine or 1-(2-hydroxy-2-methylpropyl)-1H-imidazo[4,5-c]quinolin-4-amine is between 1/1 and 4/1.

14. A method according to any preceding claim, wherein the osmolality of said solution is between 270 and 310 mOsm/kg.

**Patentansprüche für folgende Vertragsstaaten : BE, CH, DE, FR, GB, IT, LI, NL, SE**

1. Für die parenterale Verabreichung geeignete wäßrige Lösung von 1-Isobutyl-1H-imidazo[4,5-c]chinolin-4-amin oder 1-(2-Hydroxy-2-methylporpyl)-1H-imidazo[4,5 c]chinolin-4-amin, umfassend 1-Isobutyl-1H-imidazo(4,5-c)chinolin-4-amin oder 1-(2-Hydroxy-2-methylporpyl)-1H-imidazo[4,5-c]chinolin-4-amin, aufgelöst in einer wäßrigen Säure, wobei die Säure ausgewählt wird aus Salzsäure, Milchsäure, Essigsäure, Asparaginsäure, und einer Mischung von zwei oder mehreren der Vorgenannten, und ferner umfassend einen Tonusregulierer, ausgewählt aus Glycerin, Sorbitol und Dextrose in einer solchen Menge, daß die Osmolalität der Lösung zwischen 235 und 335 mOsm/kg liegt, wobei die Lösung ferner dadurch gekennzeichnet ist, daß sie einen pH-Wert zwischen 2 und 6 aufweist.

2. Lösung nach Anspruch 1, in der 1-Isobutyl-1H-imidazo[4,5-c]chinolin-4-amin oder 1-(2-Hydroxy-2-methylpropyl)-1H-imidazo[4,5-c]chinolin-4-amin in einer für den beabsichtigten therapeutischen Zweck wirksamen Menge vorliegen.

3. Lösung nach Anspruch 1 oder 2, in der die Säure Salzsäure ist.

4. Lösung nach Anspruch 3, umfassend 1-Isobutyl-1H-imidazo[4,5-c]chinolin-4-amin und bei der die Salzsäure mindestens zum Teil durch Salzsäure vorgesehen wird, die in 1-Isobutyl-1H-imidazo[4,5-c]chinolin-4-amin-Hydrochlorid vorliegt.

5. Lösung nach Anspruch 1 oder 2, in der die Säure Milchsäure ist.

6. Lösung nach einem der vorstehenden Ansprüche, bei der der pH-Wert zwischen 2,5 und 4,5 liegt.

7. Lösung nach einem der vorstehenden Ansprüche, umfassend das 1-Isobutyl-1H-imidazo[4,5-c]chinolin-4-amin oder 1-(2-Hydroxy-2-methylporpyl)-1H-imidazo[4,5 c]chinolin-4-amin in einer Menge von 0,01 bis 15 mg/ml der Lösung.

8. Lösung nach Anspruch 7, bei der 1-Isobutyl-1H-imidazo[4,5-c]chinolin-4-amin oder 1-(2-Hydroxy-2-methylpropyl)-1H-imidazo[4,5 -c]chinolin-4-amin in einer Menge von 4 bis 12 mg/ml der Lösung vorliegen.

9. Lösung nach einem der vorstehenden Ansprüche, ferner umfassend eine Base.

10. Lösung nach Anspruch 9, bei der die Base Natriumhydroxid ist.

11. Lösung nach einem der vorstehenden Ansprüche, bei der der Tonusregulierer Sorbitol ist.

12. Lösung nach einem der vorstehenden Ansprüche, bei das Molverhältnis der Säure zum 1-Isobutyl-1H-imidazo[4,5-c]chinolin-4-amin oder 1-(2-Hydroxy-2-methylporpyl)-1H-imidazo[4,5-c]chinolin-4-amin zwischen 0,5:1 und 20:1 liegt.

13. Lösung nach Anspruch 12, bei der das Molverhältnis der Säure zum 1-Isobutyl-1H-imidazo[4,5-c]chinolin-4-amin oder 1-(2-Hydroxy-2-methylporpyl)-1H-imidazo[4,5-c]chinolin-4-amin zwischen 1:1 und 4:1 liegt.

14. Lösung nach einem der vorstehenden Ansprüche, bei der die Osmolalität der Lösung zwischen 270 und 310 mOsm/kg liegt.

**Patentansprüche für folgenden Vertragsstaat : ES**

1. Verfahren zur Herstellung einer für parenterale Verabreichung geeigneten wäßrigen Lösung von 1-Isobutyl-1H-imidazo[4,5-c]chinolin-4-amin oder 1-(2-Hydroxy-2-methylpropyl)-1H-imidazo[4,5-c]chinolin-4-amin, umfassend Auflösen von 1-Isobutyl-1H-imidazo[4,5-c]chinolin-4-amin oder 1-(2-Hydroxy-2-methylpropyl)-1H-imidazo[4,5-c]chinolin-4-amin in einer wäßrigen Säure, wobei die Säure ausgewählt wird aus Salzsäure, Milchsäure, Essigsäure, Asparaginsäure, und einer Mischung von zwei oder mehreren der Vorgenannten, und weiteren Zusatz eines Tonusregulierers, ausgewählt aus Glycerin, Sorbitol und Dextrose in einer solchen Menge, daß die Osmolalität der Lösung zwischen 235 und 335 mOsm/kg liegt, wobei die Lösung auf einen pH-Wert zwischen 2 und 6 eingestellt wird.

2. Verfahren nach Anspruch 1, bei welchem 1-Isobutyl-1H-imidazo[4,5-c]chinolin-4-amin oder 1-(2-Hydroxy-2-methyl-propyl)-1H-imidazo[4,5-c]chinolin-4-amin in einer für den vorgesehenen therapeutischen Zweck ausreichenden Menge zugesetzt werden.

3. Verfahren nach Anspruch 1 oder 2 , bei welchem die Säure Salzsäure ist.

4. Verfahren nach Anspruch 3, umfassend 1-Isobutyl-1H-imidazo[4,5-c]chinolin-4-amin und bei welchem die Salzsäure mindestens zum Teil durch Salzsäure vorgesehen wird, die in 1-Isobutyl-1H-imidazo[4,5-c]chinolin-4-amin-Hydrochlorid vorliegt.

5. Verfahren nach Anspruch 1 oder 2, bei welchem die Säure Milchsäure ist.

6. Verfahren nach einem der vorstehenden Ansprüche, bei welchem der pH-Wert zwischen 2,5 und 4,5 liegt.

7. Verfahren nach einem der vorstehenden Ansprüche, umfassend das 1-Isobutyl-1H-imidazo[4,5-c]chinolin-4 -amin oder 1-(2-Hydroxy-2-methylpropyl)-1H-imidazo[4,5-c]chinolin-4-amin, die in einer Menge von 0,01 bis 15 mg/ml der Lösung zugesetzt werden.

8. Verfahren nach Anspruch 7, bei welchem 1-Isobutyl-1H-imidazo[4,5-c]chinolin-4-amin oder 1-(2-Hy- droxy-2-methyl-propyl)-1H-imidazo[4,5-c]chinolin-4-amin in einer Menge von 4 bis 12 mg/ml der Lösung vorliegen.

**9.** Verfahren nach Anspruch 1, das ferner die Zugabe einer Base umfaßt.

**10.** Verfahren nach Anspruch 9, bei welchem die Base Natriumhydroxid ist.

**11.** Verfahren nach einem der vorstehenden Ansprüche, bei welchem der Tonusregulierer Sorbitol ist.

**12.** Verfahren nach einem der vorstehenden Ansprüche, bei welchem das Molverhältnis der Säure zum 1-Isobutyl-1H-imidazo[4,5-c]chinolin-4-amin oder 1-(2-Hydroxy-2-methylpropyl)-1H-imidazo[4,5-c]chinolin-4-amin zwischen 0,5:1 und 20:1 liegt.

**13.** Verfahren nach Anspruch 2, bei welchem das Molverhältnis der Säure zum 1-Isobutyl-1H-imidazo[4,5-c]chinolin-4-amin oder 1-(2-Hydroxy-2-methylpropyl)-1H-imi-dazo[4,5-c]chinolin-4-amin zwischen 1:1 und 4:1 liegt.

**14.** Verfahren nach einem der vorstehenden Ansprüche, bei welchem die Osmolarität der Lösung zwischen 270 und 310 mOsm/kg liegt.

**Revendications**

**Revendications pour les Etats contractants suivants :BE, CH, DE, FR, GB, IT, LI, NL, SE**

**1.** Solution aqueuse de 1-isobutyl-1H-imidazo[4,5-c]quinoléin-4-amine ou 1-(2-hydroxy-2-méthylpropyl)-1H-imidazo[4,5-c]quinoléin-4-amine convenant pour l'administration parentérale, comprenant de la 1-isobutyl-1H-imidazo[4,5-c]quinoléin-4-amine ou 1-(2-hydroxy-2-méthylpropyl)-1H-imidazo[4,5-c]quinoléin-4-amine dissoute dans un acide aqueux, cet acide étant choisi parmi l'acide chlorhydrique, l'acide lactique, l'acide acétique, l'acide aspartique et les mélanges de deux de ces acides ou plus, et comprenant de plus un ajusteur de tonicité choisi parmi la glycérine, le sorbitol et le dextrose en une quantité telle que l'osmolalité de ladite solution se situe entre 235 et 335 mOsm/kg, cette solution étant en outre caractérisée en ce qu'elle a un pH se situant entre 2 et 6.

**2.** Solution suivant la revendication 1, dans laquelle la 1-isobutyl-1H-imidazo[4,5-c]quinoléin-4-amine ou 1-(2-hydroxy-2-méthylpropyl)-1H-imidazo[4,5-c]quinoléin-4-amine est présente en une quantité efficace pour l'application thérapeutique envisagée.

**3.** Solution suivant l'une ou l'autre des revendications 1 et 2, dans laquelle l'acide précité est de l'acide chlorhydrique.

**4.** Solution suivant la revendication 3, comprenant de la 1-isobutyl-1H-imidazo[4,5-c]quinoléin-4-amine et dans laquelle l'acide chlorhydrique est constitué au moins en partie par l'acide chlorhydrique présent dans du chlorhydrate de 1-isobutyl-1H-imidazo[4,5-c]quinoléin-4-amine.

**5.** Solution suivant l'une ou l'autre des revendications 1 et 2, dans laquelle l'acide précité est de l'acide lactique.

**6.** Solution suivant l'une quelconque des revendications précédentes, dans laquelle le pH se situe entre 2,5 et 4,5.

**7.** Solution suivant l'une quelconque des revendications précédentes, comprenant la 1-isobutyl-1H-imidazo[4,5-c]quinoléin-4-amine ou 1-(2-hydroxy-2-méthylpropyl)-1H-imidazo[4,5-c]quinoléin-4-amine en une quantité de 0,01 à 15 mg par millilitre de ladite solution.

**8.** Solution suivant la revendication 7, dans laquelle la 1-isobutyl-1H-imidazo[4,5-c]quinoléin-4-amine ou 1-(2-hydroxy-2-méthylpropyl)-1H-imidazo[4,5-c]quinoléin-4-amine est présente en une quantité de 4 à 12 mg par millilitre de ladite solution.

**9.** Solution suivant l'une quelconque des revendications précédentes, comprenant en outre une base.

**10.** Solution suivant la revendication 9, dans laquelle la base est de l'hydroxyde de sodium.

**11.** Solution suivant l'une quelconque des revendications précédentes, dans laquelle l'ajusteur de tonicité est du sorbitol.

**12.** Soltution suivant l'une quelconque des revendications précédentes, dans laquelle le rapport molaire de l'acide précité à la 1-isobutyl-1H-imidazo[4,5-c]quinoléin-4-amine ou 1-(2-hydroxy-2-méthyl-propyl)-1H-imidazo[4,5-c]quinoléin-4-amine se situe entre 0,5/1 et 20/1.

**13.** Solution suivant la revendication 12, dans laquelle le rapport molaire de l'acide susdit à la 1-isobutyl-1H-imidazo[4,5-c]quinoléin-4-amine ou 1-(2-hydroxy-2-méthylpropyl)-1H-imidazo[4,5-c]quinoléin-4-amine se situe entre 1/1 et 4/1.

**14.** Solution suivant l'une quelconque des revendications précédentes, dans laquelle l'osmolalité de ladite solution se situe entre 270 et 310 mOsm/kg.

## Revendications pour l'Etat contractants suivant : ES

**1.** Procédé de préparation d'une solution aqueuse de 1-isobutyl-1H-imidazo[4,5-c]quinoléin-4-amine ou 1-(2-hydroxy-2-méthylpropyl)-1H-imidazo[4,5-c]quinoléin-4-amine convenant pour l'administration parentérale, comprenant la dissolution de 1-isobutyl-1H-imidazo[4,5-c]quinoléin-4-amine ou 1-(2-hydroxy-2-méthylpropyl)-1H-imidazo[4,5-c]quinoléin-4-amine dans un acide aqueux, cet acide étant choisi parmi l'acide chlorhydrique, l'acide lactique, l'acide acétique, l'acide aspartique et les mélanges de deux de ces acides ou plus, et en outre l'addition d'un ajusteur de tonicité choisi parmi la glycérine, le sorbitol et le dextrose en une quantité telle que l'osmolalité de ladite solution se situe entre 235 et 335 mOsm/kg, cette solution étant ajustée pour avoir un pH entre 2 et 6.

**2.** Procédé suivant la revendication 1, dans lequel la 1-isobutyl-1H-imidazo[4,5-c]quinoléin-4-amine ou 1-(2-hydroxy-2-méthylpropyl)-1H-imidazo[4,5-c]quinoléin-4-amine est ajouté en une quantité efficace pour l'application thérapeutique envisagée.

**3.** Procédé suivant l'une ou l'autre des revendications 1 et 2, dans lequel l'acide précité est de l'acide chlorhydrique.

**4.** Procédé suivant la revendication 3, comprenant de la 1-isobutyl-1H-imidazo[4,5-c]quinoléin-4-amine et dans lequel l'acide chlorhydrique est constitué au moins en partie par l'acide chlorhydrique présent dans du chlorhydrate de 1-isobutyl-1H-imidazo[4,5-c]quinoléin-4-amine.

**5.** Procédé suivant l'une ou l'autre des revendications 1 et 2, dans lequel l'acide précité est de l'acide lactique.

**6.** Procédé suivant l'une quelconque des revendications précédentes, dans lequel le pH se situe entre 2,5 et 4,5.

**7.** Procédé suivant l'une quelconque des revendications précédentes, comprenant la 1-isobutyl-1H-imidazo[4,5-c]quinoléin-4-amine ou 1-(2-hydroxy-2-méthylpropyl)-1H-imidazo[4,5-c]quinoléin-4-amine en une quantité de 0,01 à 15 mg par millilitre de ladite solution.

**8.** Procédé suivant la revendication 7, dans lequel la 1-isobutyl-1H-imidazo[4,5-c]quinoléin-4-amine ou 1-(2-hydroxy-2-méthylpropyl)-1H-imidazo[4,5-c]-quinoléin-4-amine est présente en une quantité de 4 à 12 mg par millilitre de ladite solution.

**9.** Procédé suivant l'une quelconque des revendications précédentes, comprenant en outre une base.

**10.** Procédé suivant la revendication 9, dans lequel la base est de l'hydroxyde de sodium.

**11.** Procédé suivant l'une quelconque des revendications précédentes, dans lequel l'ajusteur de tonicité est du sorbitol.

**12.** Procédé suivant l'une quelconque des revendications précédentes, dans lequel le rapport molaire de l'acide précité à la 1-isobutyl-1H-imidazo-[4,5-c]quinoléin-4-amine ou 1-(2-hydroxy-2-méthyl-propyl)-1H-imi-

dazo[4,5-c]quinoléin-4-amine se situe entre 0,5/1 et 20/1.

13. Procédé suivant la revendication 12, dans lequel le rapport molaire de l'acide susdit à la 1-isobutyl-1H-imidazo[4,5-c]quinoléin-4-amine ou 1-(2-hydroxy-2-méthylpropyl)-1H-imidazo[4,5-c]quinoléin-4-amine se situe entre 1/1 et 4/1.

14. Procédé suivant l'une quelconque des revendications précédentes, dans lequel l'osmolalité de ladite solution se situe entre 270 et 310 mOsm/kg.